(19) Europäisches Patentamt
European Patent Office
Office européen des brevets

(11) Veröffentlichungsnummer : **0 085 298**
**B1**

(12) # EUROPÄISCHE PATENTSCHRIFT

(45) Veröffentlichungstag der Patentschrift : **18.06.86**

(51) Int. Cl.⁴ : **F 16 K 11/04**, F 16 K 31/06

(21) Anmeldenummer : **83100090.6**

(22) Anmeldetag : **07.01.83**

(54) **Mehrwegeventil, insbesondere zur Verwendung in Dialyse-Geräten.**

(30) Priorität : **23.01.82 DE 3202145**

(43) Veröffentlichungstag der Anmeldung : **10.08.83 Patentblatt 83/32**

(45) Bekanntmachung des Hinweises auf die Patenterteilung : **18.06.86 Patentblatt 86/25**

(84) Benannte Vertragsstaaten :
**AT BE CH DE FR GB IT LI NL SE**

(56) Entgegenhaltungen :
**CH-A- 420 762**
**DE-A- 2 134 272**
**DE-A- 2 323 528**
**US-A- 3 407 845**
**US-A- 3 525 364**
**US-A- 3 991 788**

(73) Patentinhaber : **A. und K. Müller GmbH & Co. KG**
**Bücherstrasse 31-37**
**D-4000 Düsseldorf 13 (DE)**

(72) Erfinder : **Moldenhauer, Hermann**
**Suitbertusstrasse 114**
**D-4000 Düsseldorf 1 (DE)**
Erfinder : **Müller, Kurt**
**Benrather Schlossufer 61**
**D-4000 Düsseldorf 13 (DE)**

(74) Vertreter : **Feder, Heinz, Dr. et al**
**Dominikanerstrasse 37**
**D-4000 Düsseldorf 11 (DE)**

**EP 0 085 298 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Mehrwegeventil, das insbesondere zur Verwendung in Dialyse-Geräten geeignet sein soll.

An ein solches Ventil werden hinsichtlich der Funktionsfähigkeit und der Sicherheit im Betrieb außerordentlich hohe Anforderungen gestellt.

Weiterhin soll ein derartiges Ventil noch folgenden Anforderungen genügen :

1. Das zu steuernde Medium soll nicht mit ferromagnetischen Werkstoffen in Berührung kommen, da diese als Magnetfallen für in dem Medium gelöste oder mitgeführte Stoffe wirken können.

2. Es sollen, soweit irgend möglich, nur hochwertige Kunststoffe, beispielsweise Elastomere, den Mediumsstrom tangieren.

3. Das Ventil soll möglichst wenig ungespülte Toträume aufweisen.

4. Die Funktionsweise des Ventils soll möglichst unabhängig vom herrschenden Mediumsdruck sein.

5. Der Schaltzustand des Ventils soll durch ein potentialfreies, elektrisch auswertbares Signal angezeigt werden können.

Die Erfindung geht aus von einem Mehrwegeventil mit einem Ventilkörper, durch den Zufluß- und Abflußkanäle geführt sind und an dem zwei Membranen mit gleicher Wirkungsfläche so mit ihren Rändern befestigt sind, daß jeweils zwischen einer Oberfläche einer der Membranen und einer Oberfläche des Ventilkörpers eine Ventilkammer gebildet wird und an mindestens einer der Membranen ein Ventilteller angeordnet ist, der einem in mindestens einer der Ventilkammern angeordneten Ventilsitz gegenüberliegt, über welchen diese Ventilkammer mit einem Abflußkanal verbindbar ist und beide Membranen über eine in Bewegungsrichtung der Membranen starre, mechanische Kopplungsvorrichtung miteinander so verbunden sind, daß sich die Membranen in Bezug auf die Ventilkammern gegensinnig bewegen und eine Betätigungsvorrichtung zur Bewegung der Membranen vorhanden ist.

Ein derartiges Ventil ist bekannt und beispielsweise in der DE-A-2 134 272 beschrieben.

Dieses bekannte Ventil hat den Nachteil, daß mit ihm nicht alle der oben erwähnten Forderungen erfüllbar sind. So ist beispielsweise die Kopplungsvorrichtung zwischen den beiden Ventiltellern, die jeweils an den Enden von Faltenbälgen angeordnet sind, durch einen gemeinsamen Ventilsitz hindurchgeführt und liegt damit im Mediumsstrom. Es ist aber außerordentlich schwierig, eine solche Kopplungsvorrichtung, die unter Umständen erhebliche Kräfte zu übertragen hat, aus nichtmetallischem Werkstoff oder gar aus Kunststoff herzustellen. Eine gewisse Unabhängigkeit vom Mediumsdruck wird bei dem bekannten Ventil dadurch erreicht, daß die Ventilteller als Ringe ausgebildet und die Faltenbälge offen sind. Dies hat aber den Nachteil, daß erhebliche ungespülte Toträume entstehen.

In der US-A-3 407 845 ist ein Mehrwegeventil beschrieben, bei dem zwei Zuflußkanäle über jeweils einander gegenüberliegende Ventilsitze mit einem Abflußkanal verbindbar sind. Die den Ventilsitzen gegenüberliegenden Ventilteller sind jeweils in eine Membran integriert und durch einen gemeinsamen Ventilschaft miteinander gekoppelt. Dieser Ventilschaft ist jedoch durch die Ventilsitze hindurchgeführt und liegt damit direkt im Mediumsstrom.

In der CH-A-420 762 ist ein Mehrwegemagnetventil beschrieben, bei dem der eine Leitungsanschluß über einander gegenüberliegende Ventilsitze mit den anderen beiden Leitungsanschlüssen verbunden ist. Zwischen den beiden Ventilsitzen befindet sich ein Doppelsitzverschlußstück aus Kunststoff, dessen Betätigungsgestänge durch einen Ventilsitz hindurchgeführt ist. Dieses Betätigungsgestänge ist von einem schlauchartigen Ansatz aus Kunststoff umgeben, der einerseits mit dem Doppelsitzverschlußstück und andererseits mit dem Gehäuse verbunden ist, so daß das Medium nicht in direkten Kontakt mit dem Betätigungsgestänge gerät. Da das Ventilgehäuse ebenfalls aus Kunststoff bestehen kann, kommt bei diesem Ventil das Medium nur mit Kunststoffteilen in Berührung. Die Führung des Betätigungsgestänges durch den Ventilsitz hat aber den Nachteil, daß eine Flächendifferenz zwischen der Nennweite des Ventils und der Wirkungsfläche auftritt, die eine Erhöhung der vom Elektromagneten aufzubringenden Stellkraft erfordert. Dies ist vor allem bei der Ausführungsform mit kleinen Nennweiten sehr nachteilig, da das Betätigungsgestänge nicht beliebig dünn ausgebildet werden kann. Außerdem ist es schwierig, dieses Ventil so auszugestalten, daß es völlig unabhängig vom Systemdruck ist.

In der US-A-3 991 788 ist ein Mehrwegeventil beschrieben, bei dem in eine gemeinsame Membran mehrere Ventilteller integriert sind, die jeweils Ventilsitzen gegenüberliegen, durch welche Abflußkanäle mit einer gemeinsamen Ventilkammer verbindbar sind. An der Rückseite der Membran sind Ventilstößel angeordnet, die mit ihren vorderen Enden an der Rückseite der Ventilteller anliegen, aber nicht fest mit diesen verbunden sind. Auf diese Weise sind die Ventilsitze nicht starr miteinander gekoppelt, was zur Folge hat, daß die Funktionsweise des Ventils nicht unabhängig vom Systemdruck ist.

Die der Erfindung zugrunde liegende Aufgabe bestand darin, ein Ventil der oben und im Oberbegriff des Patentanspruchs 1 angegebenen Bauart so auszugestalten, daß mit ihm die oben aufgestellten Forderungen verwirklichbar sind. Weiterhin sollte das Ventil ohne größere Abänderungen der Grundkonstruktion als 3/2-Wegeventil oder als entweder im Ruhezustand geschlossenes oder im Ruhezustand offenes 2/2-Wegeventil ausgebildet werden können. Schließlich sollten bei der Ausbildung des Ventils als Elektromagnetventil bei möglichst hohen hydraulischen Leistungen die elektrischen Werte möglichst niedrig liegen.

Die Lösung dieser Aufgabe geschieht erfindungsgemäß dadurch, daß die beiden Ventilkammern miteinander und mit einem gemeinsamen Zuflußkanal verbunden sind und sowohl die Membranen als auch der bzw. die Ventilteller geschlossen ausgebildet sind und jeder Ventilteller einstückig in die ihm zugeordnete Membran integriert ist und die Kopplungsvorrichtung außerhalb der Ventilkammer und der Zufluß- und Abflußkanäle angeordnet ist.

Wie weiter unten anhand von Ausführungsbeispielen genauer erläutert werden wird, erfüllt das erfindungsgemäße Ventil in hervorragender Weise die oben aufgestellten Forderungen. Die Unabhängigkeit vom Systemdruck wird dadurch erreicht, daß die beiden Membranen in gleichem Maße vom Systemdruck beaufschlagt werden, der durch die starre mechanische Kopplungsvorrichtung kompensiert wird. Es ist grundsätzlich möglich, das erfindungsgemäße Ventil unter Verwendung von Flachmembranen auszubilden. Es hat sich aber als besonders vorteilhaft erwiesen, wenn die Membranen entweder als Rollmembranen oder als Faltenbälge ausgebildet sind, da bei diesen Membrantypen bei einer Ausdehnung keinerlei Änderung der Wirkungsfläche auftritt und somit eine vollkommene Kompensierung des Systemdrucks möglich ist, so daß die erforderliche Stellkraft nur noch von den Flächendifferenzen der Ventilsitze bestimmt ist.

Dies bedeutet, daß dei einem Ventil der Nennweite 3 mm in jeder Schaltstellung die Kopplungsvorrichtung mit den beiden Membranen mit einer Kraft von 0,7 N/bar in der Endlage gehalten wird. Die Wirkungsweise des Ventils ist daher als bistabil anzusehen.

Verschiedene vorteilhafte Ausführungsformen des erfindungsgemäßen Ventils sind möglich und weiter unten sowie in den Unteransprüchen beschrieben.

Das erfindungsgemäße Ventil kann als Elektromagnetventil ausgebildet sein, wobei die Betätigungsvorrichtung eine Magnetspule mit einem gegen Federkraft bewegbaren Anker enthält. Es können aber auch andere Betätigungsvorrichtungen, beispielsweise hydraulisch oder pneumatisch betätigte Betätigungsvorrichtungen verwendet werden.

So kann beispielsweise die Betätigungsvorrichtung auch einen bewegten zwei stabile Endlagen aufweisenden Dauermagneten enthalten, sowie eine mit Schaltimpulsen beaufschlagbare Magnetspule zur Beförderung des Dauermagneten aus der einen in die andere Endlage. Da der magnetische Kreis vollkommen vom hydraulischen Kreis getrennt ist, ist der Einsatz eines derartigen Dauermagneten möglich, weil auf die Korrosionsbeständigkeit keine Rücksicht genommen werden muß.

Da die aktiven Betätigungsmechanismen des erfindungsgemäßen Ventils von außen frei zugänglich sind, ist es weiterhin in einfacher Weise möglich, diese Betätigungsmechanismen, beispielsweise die Kopplungsvorrichtung, durch entsprechende Vorrichtungen abzutasten, zur Feststellung des Schaltzustandes des Ventils. Eine derartige Abtastung kann beispielsweise durch Näherungsinitiatoren oder auch einen einfachen Mikroschalter erfolgen.

Bei einer Ausbildung des erfindungsgemäßen Ventils als Elektromagnetventil ist auch eine Betätigung mittels Wechselstrom ohne weiteres durchführbar, da die Differenz zwischen Kraft- und Formschluß durch das elastische Verhalten der in die Membranen integrierten Ventilteller überbrückt wird.

Wie weiter unten näher erläutert, braucht die in Bewegungsrichtung der Membranen starre, mechanische Kopplungsvorrichtung nicht notwendig aus Festkörperbauteilen aufgebaut zu sein, sondern es ist möglich, hier ein hydraulisches System zu verwenden.

Im folgenden werden anhand der beigefügten Zeichnung Ausführungsbeispiele des erfindungsgemäßen Ventils näher erläutert.

In den Zeichnungen zeigen :

Figur 1 im Längsschnitt eine erste Ausführungsform des erfindungsgemäßen Ventils in einer Ausbildung als 3/2-Wegeventil ;

Figur 2 eine Variante des Ventils nach Fig. 1 in einer Ausbildung als im Ruhezustand offenes 2/2-Wegeventil ;

Figur 3 eine weitere Variante des Ventils nach Fig. 1 in einer Ausbildung als im Ruhezustand geschlossenes 2/2-Wegeventil ;

Figur 4 eine zweite Ausführungsform des erfindungsgemäßen Ventils in einer Ausbildung als 3/2-Wegeventil ;

Figur 5 eine dritte Ausführungsform des erfindungsgemäßen Ventils in einer Ausbildung als 3/2-Wegeventil ;

Figur 6 eine vierte Ausführungsform des erfindungsgemäßen Ventils in einer Ausbildung als 3/2-Wegeventil mit einer als hydraulisches System ausgebildeten Kopplungsvorrichtung ;

Figur 7 eine weitere Variante des Ventils nach Fig. 1 in einer Ausbildung als 3/2-Wegeventil mit sich überschneidenden Schließstellungen ;

Figur 8 eine Teilansicht einer Variante der Ausführungsformen nach den Fig. 1 bis 5 für ein bei Systemunterdruck verwendbares Ventil.

In Fig. 1 ist ein 3/2-Wegeventil dargestellt, das als Elektromagnetventil ausgebildet ist. Es weist einen als beispielsweise rechteckiger Block ausgebildeten Ventilkörper 1 auf, durch den ein Zuflußkanal 2 und zwei Abflußkanäle 3a, 3b geführt sind. Der Ventilkörper 1 kann beispielsweise aus Kunststoff oder einem anderen nichtferromagnetischem Material bestehen. An der in Fig. 1 oberen Seite des Ventilkörpers 1 sind die Ränder einer Rollmembran 4 befestigt, deren Innenseite zusammen mit den entsprechenden Flächen des Ventilkörpers 1 eine erste Ventilkammer 6 bildet. An der in Fig. 1 unteren Seite des Ventilkörpers 1 ist in der gleichen Weise eine Rollmembran 5 befestigt, die mit der Oberfläche des Ventilkörpers eine zweite Ventilkammer 7 bildet. Die Ventilkammern 6 und 7 sind durch einen Verbindungskanal 2a miteinander verbunden, in den auch der Zuflußkanal 2 einmündet. Die Abflußkanäle 3a und 3b münden in Ventilsitze 6a bzw. 7a ein, die in den Ventilkammern 6 und 7 angeordnet sind. Die sich an den Abstützflächen 4b bzw. 5b abrollenden Rollmembranen 4 und 5 sind in ihrem mittleren Bereich als Ventilteller 4a bzw. 5a ausgebildet, wobei der Ventilteller 4a dem Ventilsitz 6a und der Ventilteller 5a dem Ventilsitz 7a gegenüberliegt. Die Rollmembranen 4 und 5 Können aus Kunststoff, Gummi oder einem anderen geeigneten elastischen Material hergestellt sein. Die Ventilteller 4a und 5a sind einstückig in die Rollmembranen 4 und 5 integriert. Die mittleren Bereiche der beiden Rollmembranen 4 und 5 sind über einen rechteckigen, die Ventilkammern 6, 7 umfassenden insgesamt mit Bezugsziffer 7 bezeichneten Rahmen miteinander verbunden. Der Rahmen besteht aus zwei quer zur Längsachse L des Ventils verlaufenden Teilen 8a und zwei parallel zur Längsachse L des Ventils verlaufenden, als Führungsstangen ausgebildeten Teilen 8b. Die Teile 8a sind über Verbindungsstücke 8c mit den Membranen 4 bzw. 5 verbunden. Die Teile 8b sind in Ausnehmungen oder Bohrungen 8d geführt, die im Ventilkörper 1 angeordnet sind. Die Kopplung der beiden Membranen 4 und 5 durch den Rahmen 8 erfolgt so, daß jeweils einer der beiden Ventilteller (in Fig. 1 Ventilteller 4a) auf dem Ventilsitz (in Fig. 1 Ventilsitz 6a) aufsitzt, während der jeweils andere Ventilteller (in Fig. 1 Ventilteller 5a) vom Ventilsitz (in Fig. 1 Ventilsitz 7a) abgehoben ist.

Der Ventilkörper 1 ist über einen Halterungsrahmen 9 mit einer Betätigungsvorrichtung verbunden, die in der dargestellten Ausführungsform eine Magnetspule 10 aufweist, welche von einem Joch 11 umfaßt ist und in der ein bewegbarer Anker 12 geführt ist, der an seinem unteren Ende mit dem Rahmen 8 verbunden ist. Im Ruhezustand des Ventils wird der Anker 12 durch die Kraft einer Feder 13 nach unten gedrückt, so daß der in Fig. 1 dargestellte Zustand des Ventils eintritt, bei dem der Ventilsitz 6a geschlossen und der Ventilsitz 7a geöffnet ist. Bei Erregung der Magnetspule 10 wird der Anker 12 gegen die Kraft der Feder 13 angezogen und dadurch der Ventilsitz 6a geöffnet und der Ventilsitz 7a geschlossen.

Wie aus Fig. 1 unmittelbar abzulesen, wirkt der Systemdruck über den Zuflußkanal 12 gleichmäßig auf die Innenseite der beiden Membranen 4 und 5 und dieser Druck wird durch den die beiden Membranen verbindenden Rahmen 8 kompensiert. Der Rahmen 8 durchdringt an keiner Stelle Hohlräume, die vom Mediumsstrom durchflossen sind. Das Ventil weist außer den beiden Ventilkammern 6 und 7 sowie den Zuflußkanälen 2 und 2a und den Abflußkanälen 3a und 3b keine weiteren vom Medium beaufschlagten Hohlräume auf. Sämtliche vom Medium durchströmten Teile können aus Kunststoff hergestellt sein.

Bei der in Fig. 1 dargestellten Ausführungsform ist der Ventilkörper 1 als geschlossener Block dargestellt. Selbstverständlich sind auch andere Ausführungsformen möglich, bei denen die Zuflußkanäle 2 und 2a und die Abflußkanäle 3a und 3b als Rohre durch einen beispielsweise als Halterungsrahmen ausgebildeten Ventilkörper geführt sind, der nur an der den Ventilkammer 6 und 7 zugewandten Seiten geschlossene Flächen aufweist.

In Fig. 2 ist eine Variante der Ausführungsform nach Fig. 1 dargestellt, die als 2/2-Wegeventil ausgebildet ist, und zwar in der Weise, daß das Ventil im Ruhezustand geöffnet ist. In Fig. 2 sind gleich ausgebildete Bauteile mit gleichen Bezugsziffern wie Fig. 1 versehen. Durch den ähnlich wie in Fig. 1 ausgebildeten Ventilkörper 1' ist ein Zuflußkanal 2' und ein Abflußkanal 3a' geführt. An den beiden voneinander abgewandten Flächen des Ventilkörpers 1' sind analog der Ausführungsform nach Fig. 1 Rollmembranen 4, 5 angeordnet, die sich an Abrollflächen 4b, 5b abstützen und in der Mitte als Ventilteller 4a, 5a ausgebildet sind. Die Rollmembranen 4, 5 begrenzen zusammen mit dem Ventilkörper 1' die Ventilkammern 6, 7. Die Ventilkammer 6 ist durch den Ventilkörper 1' hindurch über einen Verbindungskanal 2a' mit der Ventilkammer 7 verbunden. Der Zuflußkanal 2' mündet in die Ventilkammer 6, während der Abflußkanal 3a' über den Ventilsitz 7a' in die Ventilkammer 7 mündet. In der Ventilkammer 6 ist an der Stelle des Ventilsitzes lediglich ein Aufsatz 6a' angeordnet, auf dem der Ventilteller 4a in der in Fig. 2 dargestellten Stellung aufsitzt. Die beiden Membranen 4 und 5 sind wiederum durch den aus den Teilen 8a, 8b und 8c bestehenden Rahmen 8 miteinander verbunden, wobei die Teile 8b durch die Ausnehmungen 8d im Ventilkörper 1' hindurchtreten. Der Ventilkörper 1' ist über dem Halterungsrahmen 9 mit der die Magnetspule 10, das Joch 11 und den gegen die Feder 13 bewegbaren Anker 12 enthaltenden Betätigungsvorrichtung verbunden.

Der Systemdruck wirkt in beiden Schaltstellungen des Ventils über den Zuflußkanal 2' auf die Membran 4 und den Verbindungskanal 2a' auf die Membran 5. Im geöffneten Zustand des Ventils werden die beiden Ventilkammern 6 und 7 vom Medium durchströmt. Beim Anziehen des Ankers 12 gegen die Kraft der Feder 13 legt sich der Ventilteller 5a auf den Ventilsitz 7a' auf und das Ventil schließt.

Die in Fig. 3 dargestellte Ausführungsform unterscheidet sich von der in Fig. 2 dargestellten Ausführungsform in einigen Einzelheiten, durch die das Ventil zu einem im Ruhezustand geschlossenen 2/2-Wegeventil wird. Der Ventilkörper 1" enthält den in die untere Ventilkammer 7 mündenden Zuflußkanal 2" sowie den in die obere Ventilkammer 6 mündenden Abflußkanal 3a". Die beiden Ventilkammern 6 und 7 sind wiederum durch einen den Ventilkörper 1" durchdringenden Verbindungskanal 2a" miteinander verbunden. In der oberen

Ventilkammer 6 befindet sich der mit dem Abflußkanal 3a″ verbundene Ventilsitz 6a″, während in der unteren Ventilkammer 7 an der gleichen Stelle ein Aufsatz 7a″ angeordnet ist, auf den sich der Ventilteller 5a bei geöffnetem Ventil aufsetzt. Die beiden Rollmembranen 4, 5 sind wiederum über den anhand von Fig. 1 und 2 beschriebenen Rahmen 8 miteinander verbunden. Die über den Halterungsrahmen 9 mit dem Ventilkörper 1″ verbundene Betätigungsvorrichtung weist wiederum eine Magnetspule 10 mit Joch 11 sowie einen gegen die Wirkung der Feder 13 bewegbaren Anker 12 auf.

Wie aus Fig. 3 unmittelbar abzulesen, liegt in dem in Fig. 3 dargestellten Ruhezustand der Ventilteller 4a auf dem Ventilsitz 6a″ auf und das Ventil ist geschlossen. Beim Anziehen des Ankers 12 gegen die Kraft der Feder 13 öffnet das Ventil.

Um einen guten Ausgleich des Systemdrucks zu erzielen, können bei den Ausführungsformen nach Fig. 2 und 3 anstelle des einen Verbindungskanals 2a′ und 2a″ auch mehrere Verbindungskanäle vorhanden sein, die um den Ventilsitz 7a′ bzw. 6a″ herum angeordnet sein können.

In Fig. 4 ist eine andere Ausführungsform eines 3/2-Wegeventils dargestellt, bei der sich die Ventilkammern auf der gleichen Seite eines Ventilkörpers befinden.

Um den Zusammenhang mit den vorbeschriebenen Ausführungsformen aufzuzeigen, sind auch hier Bauteile, die in ihrer Ausbildung genau Bauteilen der Ausführungsform nach Fig. 1 entsprechen, mit gleichen Bezugsziffern bezeichnet.

Der wiederum als flaches Bauteil ausgebildete Ventilkörper 21 ist von einem gemeinsamen Zuflußkanal 22 und den beiden Abflußkanälen 23a und 23b durchdrungen. An der in Fig. 4 oberen Seite des Ventilkörpers 21 sind die Rollmembranen 4 und 5 angeordnet, die jeweils zusammen mit dem entsprechenden Teil dieser Oberfläche des Ventilkörpers 21 die Ventilkammern 6 und 7 bilden. Die Ventilkammern 6 und 7 sind durch einen Verbindungskanal 22a verbunden, in den der gemeinsame Zuflußkanal 22 einmündet. In die Ventilkammer 6 mündet der Abflußkanal 23a über den Ventilsitz 6a und in die Ventilkammer 7 mündet der Abflußkanal 23b über den Ventilsitz 7a. Die sich an den Widerlagern 4b bzw. 5b abstützenden Rollmembranen 4, 5 weisen in ihrem mittleren Bereich die integrierten Ventilteller 4a und 5a auf, die den Ventilsitzen 6a und 7a gegenüberliegen. Die Außenseiten der Rollmembranen 4 und 5 sind in ihrer Mitte über eine Koppelungsvorrichtung 28 verbunden, die in diesem Falle als zweiarmiger Hebel ausgebildet ist, der sich mit seinem Drehpunkt 28d am Ventilkörper 21 abstützt und dessen einer Hebelarm 28a über ein Verbindungsstück 28c mit der Rollmembran 4 und dessen anderer Hebelarm 28b über ein Verbindungsstück 28c mit der Rollmembran 5 verbunden ist. Durch diese Art der Koppelung ist, wie bei den vorbeschriebenen Ausführungsformen sichergestellt, daß die Bewegung der beiden Rollmembranen 4 und 5 in Bezug auf die Ventilkammern 6 und 7 gegensinnig erfolgt. Oberhalb des Ventilkörpers 21 ist über eine Halterungsrahmen 29 eine Betätigungsvorrichtung angeordnet, die eine Magnetspule 10 mit Joch 11 enthält, in der ein Anker 12′ gegen die Kraftwirkung einer Feder 13′ geführt ist. das äußere Ende des Ankers 12′ ist über ein Verbindungselement 27 mit dem Verbindungsstück 28c am Hebelarm 28a verbunden. Beim Anziehen des Ankers 12′ hebt der Ventilteller 4a vom Ventilsitz 6a ab und der Ventilteller 5a setzt auf den Ventilsitz 7a auf.

Die Funktionsweise des in Fig. 4 dargestellten Ventils ist grundsätzlich die gleiche wie bei der Ausführungsform nach Fig. 1. Der Systemdruck wirkt über den Zuflußkanal 22 und den Verbindungskanal 22a auf die Innenseite beider Rollmembranen 4, 5 und wird durch den Hebel 28 kompensiert.

Auch die in Fig. 4 dargestellte Ausführungsform läßt sich als 2/2-Wegeventil ausbilden, in dem einer der beiden Abflußkanäle geschlossen wird und dafür in die Ventilkammer, in der der Abfluß geschlossen ist, der Zufluß direkt hineingeführt wird, während der Verbindungskanal erhalten bleibt.

In Fig. 5 ist eine als 3/2-Wegeventil ausgebildete Ausführungsform dargestellt, bei der die Betätigungsvorrichtung zwischen beiden Ventilkammern angeordnet ist. Auch bei dieser Ausführungsform sind die der Ausführungsform nach Fig. 1 genau entsprechenden Bauteile mit gleichen Bezugsziffern bezeichnet.

Der Ventilkörper 31 ist bei dieser Ausführungsform U-förmig ausgebildet mit den beiden Schenkeln 31 a und 31b. An den einander zugewandten Flächen der beiden Schenkel 31a und 31b sind die Rollmembranen 4 und 5 angeordnet, die mit Teilen dieser Innenflächen die Ventilkammern 6 und 7 bilden. Diese sind über einen durch den Ventilkörper 1 hindurchlaufenden Verbindungskanal 32a, in den auch der Zuflußkanal 32 einmündet, miteinander verbunden. In die Ventilkammer 6 mündet ein durch das Bauteil 31a geführter Abflußkanal 33a, während in die Ventilkammer 7 ein durch das Bauteil 31b hindurchgeführter Abflußkanal 33b einmündet. Die Rollmembran 4 weist, wie bei den vorbeschriebenen Ausführungsformen einen dem Ventilsitz 6a gegenüberliegenden Ventilteller 4a auf, während die Rollmembran 5 den dem Ventilsitz 7a gegenüberliegenden Ventilteller 5a aufweist.

Die beiden Rollmembranen 4 und 5 sind über eine Kopplungsvorrichtung miteinander verbunden, welche durch die zwischen den beiden Schenkeln 31a und 31b angeordnete Betätigungsvorrichtung hindurchgeführt ist. Diese Betätigungsvorrichtung besitzt, wie bei den vorbeschriebenen Ausführungsformen, eine über eine Halterung 39 mit dem Ventilkörper 31 verbundene Magnetspule 10 mit Joch 11, in der ein Anker 12″ gegen die Kraft einer Feder 13″ bewegbar geführt ist. Das eine Ende des Ankers 12″ ist über eine in einer Führung 38b geführte Verbindungsstange 38a und ein Verbindungsstück 38c mit der Membran 4 verbunden, während das andere Ende des Ankers direkt mit der Membran 5 verbunden ist. In dem in Fig. 5 dargestellten Schaltzustand des Ventils, der dem Ruhezustand der Betätigungsvorrichtung entspricht, sitzt der Ventilteller 5a auf dem Ventilsitz 7a auf. Beim Anziehen des Ankers 12" hebt der Ventilteller 5a vom Ventilsitz 7a ab und der Ventilteller 4a setzt sich auf den Ventilsitz 6a auf.

Auch bei dieser Ausführungsform sind alle der eingangs aufgestellten Forderungen erfüllt. Weiterhin kann auch diese Ausführungsform durch kleinere Umbauten in ein 2/2-Wegeventil umgewandelt werden.

In Fig. 6 ist eine als 3/2-Wegeventil ausgebildete Ausführungsform dargestellt, die bezüglich der Anordnung der Ventilkammern am Ventilkörper im wesentlichen der Ausführungsform nach Fig. 1 entspricht. Die Kopplung der beiden Rollmembranen 4 und 5 erfolgt bei dieser Ausführungsform nicht über ein mit festen Bauteilen aufgebautes mechanisches System, sondern über ein hydraulisches System.

Durch den Ventilkörper 41 sind der in den Verbindungskanal 42a der beiden Ventilkammern 6 und 7 einmündende Zuflußkanal 42, sowie die über die Ventilsitze 6a, 7a in die Ventilkammern 6 und 7 einmündenden Abflußkanäle 43a und 43b geführt. Die beiden Rollmembranen 4 und 5 sind an ihrer Außenseite mit Hydraulikzylindern verbunden, und zwar der Gestalt, daß die Widerlager der Rollmembranen 4 und 5 gleichzeitig als Zylinder 44 und 45 ausgebildet sind, in denen jeweils ein mit der Rollmembran verbundener Kolben 46 bzw. 47 geführt ist. Die Hydraulikflüssigkeit wird demnach direkt auf die Außenseite der Rollmembranen 4, 5 und auf die Flächen der mit ihnen verbundenen Kolben 46, 47. Die Hydraulikzylinder 44, 45 sind über eine aus den Zweigen 48 und 49 bestehende Hydraulikleitung direkt miteinander verbunden. Die beiden Hydraulikleitungen 48, 49 münden an beiden Enden in einen Steuerzylinder 50 ein, in dem ein Steuerkolben 52 gegen die Kraft einer Feder 53 verschiebbar geführt ist. Sowohl die beiden Hydraulikzylinder 4 und 45 als auch die Hydraulikleitungen 48 und 49 und die an die Stirnflächen des Steuerkolbens 52 angrenzenden Steuerräume 51a und 51b sind ganz mit der Hydraulikflüssigkeit gefüllt. Der Steuerkolben 52 ist gleichzeitig als Anker einer Magnetspule 10 mit Joch 11 ausgebildet. An der einen Stirnfläche des Steuerkolbens 52 ist ein Ventilteller 54a angeordnet, der einem Ventilsitz 55a gegenüberliegt, während an der anderen Stirnfläche des Steuerkolbens 52 ein Ventilteller 54b angeordnet ist, der einem Ventilsitz 55b gegenüberliegt. Gegen die Wand des Steuerzylinders 50 ist der Steuerkolben 52 durch eine Ringdichtung 56 abgedichtet, welche einen Durchtrittsschlitz 56a aufweist. In dem in Fig. 6 dargestellten Zustand, der dem Ruhezustand der Betätigungsvorrichtung entspricht, sitzt der Ventilteller 4a auf dem Ventilsitz 6a auf, während der Ventilteller 5a vom Ventilsitz 7a abgehoben ist. Beim Anziehen des den Steuerkolben 52 bildenden Ankers gegen die Kraft der Federn 53 wird die hydraulische Flüssigkeit über die Leitung 49 in den Hydraulikzylinder 45 gedrückt und aus dem Hydraulikzylinder 44 über die Leitung 48 abgezogen. Wegen der Inkompressibilität der Hydraulikflüssigkeit und dem völlig gefüllten Zustand des Systems entsteht eine in Bewegungsrichtung der Rollmembranen 4 und 5 starre Verbindung zwischen den beiden Rollmembranen und der Ventilteller 4a hebt ab, während der Ventilteller 5a aufsetzt.

In den beiden Endstellungen des Steuerkolbens 52 ist der hydraulische Kreis durch die Ventile 54a-55a bzw. 54b-55b gesperrt, wodurch die festen Endlagen der Ventilteller 4a und 5a sichergestellt sind. Ein statischer Druckausgleich zwischen den beiden Leitungen 48, 49 erfolgt über einen Durchtrittsschlitz 56a im Dichtungsring 56.

Die in Fig. 6 dargestellte Ausführungsform hat den großen Vorteil, daß Ventil und Betätigungsvorrichtung räumlich getrennt angeordnet werden können.

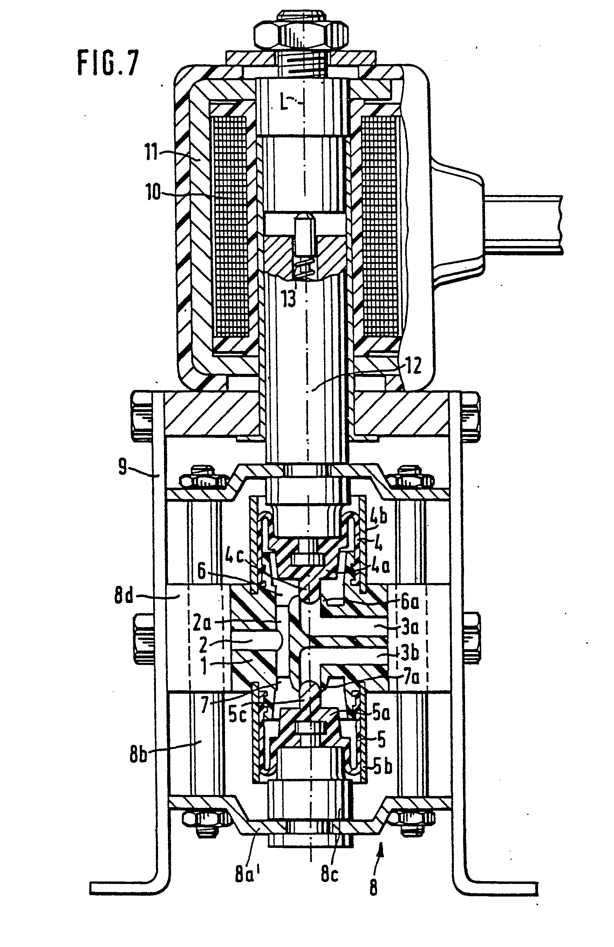

In Fig. 7 ist eine Variante der Ausführungsform nach Fig. 1 dargestellt, die als 3/2-Wegeventil ausgebildet ist und die sich von der Ausführungsform nach Fig. 1 dadurch unterscheidet, daß an jedem der beiden Ventilteller 4a und 5a an der jeweils dem Ventilsitz 6a und 7a zugewandten Seite ein Verschlußstopfen 4c und 5c angeordnet ist, dessen Durchmesser mindestens annähernd dem Innendurchmesser der Ventilsitze 6a und 7a entspricht. Die Länge dieser Verschlußstopfen 4c und 5c ist derart bemessen, daß in einer in Fig. 7 dargestellten mittleren Stellung des Ventils beide Verschlußstopfen 4c und 5c in den jeweiligen Ventilsitz 6a und 7a eintauchen und diesen dadurch verschließen. Auf diese Weise wird erreicht, daß in einer mittleren Stellung des Ventils die beiden Abflußkanäle 3a und 3b verschlossen sind. Der Gesamthub der Ventilteller muß so bemessen sein, daß in den beiden Endstellungen jeweils einer der beiden Ventilsitze 6a oder 7a geöffnet ist. Dies bedeutet, daß der Gesamthub größer sein muß als beispielsweise bei der Ausführungsform nach Fig. 1, und zwar um eine Strecke, die der Länge der Verschlußstopfen 4c bzw. 5c entspricht. Der gegenüber der Ausführungsform nach Fig. 1 etwas größere erforderliche Hub ist bei der Ausführungsform nach Fig. 7 konstruktiv berücksichtigt, in dem das in Querrichtung verlaufende Teil 8a' des Rahmens 8, wie aus der Fig. ersichtlich, gekröpft ausgebildet ist.

Die Ausführungsform nach Fig. 7 ist dann von besonderer Bedeutung, wenn im Betrieb des Ventils vermieden werden soll, daß beim Umschalten in einer Mittelstellung für einen kurzen Augenblick die beiden Abflußkanäle 3a und 3b geöffnet sind, was zu Fehlern bei der Dosierung der gesteuerten Flüssigkeitsmenge führen kann. Bei der Ausführungsform nach Fig. 7 tritt also eine Überschneidung der Schließphasen auf.

Bei den in den Fig. 1 bis 5 dargestellten Ausführungsformen des Ventils sind die Rollmembranen 4 bzw. 5 so angeordnet, daß ihre Falten jeweils nach Außen weisen. In dieser Ausführungsform ist das Ventil für eine Verwendung bei Systemüberdruck gedacht. Es ist selbstverständlich auch möglich, die Ventile bei innerem Systemunterdruck zu verwenden. In diesem Falle muß allerdings bei der Verwendung von Rollmembranen die Einbaulage der Rollmembranen umgekehrt werden. Wie dies zu geschehen hat, ist in Fig. 8 dargestellt. Fig. 8 zeigt einen Ausschnitt eines Ventils einer Ausführungsform, wie sie den Fig. 1 bis 5 entsprechen kann, im Bereich einer Rollmembran. Die Rollmembran 4' ist am Ventilkörper 1 in der Weise befestigt, daß ihre abrollende Außenseite sich an mit dem Ventilkörper 1 verbundenen Abstützflächen 4b' abstütz. Wie bei den anderen Ausführungsformen ist die Rollmembran 4' in ihrem mittleren Bereich als Ventilteller 4a' ausgebildet, der dem Ventilsitz 6a gegenüberliegt.

**Patentansprüche**

1. Mehrwegeventil, insbesondere zur Verwendung in Dialyse-Geräten, mit einem Ventilkörper, durch den Zufluß- und Abflußkanäle (2, 22, 32, 42 bzw. 3a, 3b, 23a, 23b, 33a, 33b, 43a, 43b) geführt sind, und an dem zwei Membranen (4, 5) mit gleicher Wirkungsfläche so mit ihren Rändern befestigt sind, daß jeweils zwischen einer Oberfläche einer der Membranen und einer Oberfläche des Ventilkörpers eine Ventilkammer (6, 7) gebildet wird und an mindestens einer der Membranen ein Ventilteller (4a, 5a) angeordnet ist, der einem in mindestens einer der Ventilkammern angeordneten Ventilsitz (6a, 7a) gegenüberliegt, über welchen diese Ventilkammer mit einem Abflußkanal verbindbar ist und beide Membranen über eine in Bewegungsrichtung der Membranen starre mechanische Kopplungsvorrichtung (8, 28, 38a, 48 bis 52) miteinander so verbunden sind, daß sich die Membranen in Bezug auf die Ventilkammern gegensinnig bewegen und eine Betätigungsvorrichtung (10) zur Bewegung der Membranen vorhanden ist, dadurch gekennzeichnet, daß die beiden Ventilkammern (6, 7) miteinander und mit einem gemeinsamen Zuflußkanal (2, 22, 32, 42) verbunden sind und sowohl die Membranen (4, 5) als auch der bzw. die Ventilteller (4a, 5a) geschlossen ausgebildet sind und jeder Ventilteller (4a, 5a) einstückig in die ihm zugeordnete Membran integriert ist und die Kopplungsvorrichtung (8, 28, 38a, 48 bis 52) außerhalb der Ventilkammern (6, 7) und der Zufluß- und Abflußkanäle (2, 22, 32, 42 bzw. 3a, 3b, 23a, 23b, 33a, 33b, 43a, 43b) angeordnet ist.

2. Mehrwegeventil nach Anspruch 1, dadurch gekennzeichnet, daß die Membranen als Rollmembranen (4, 5) ausgebildet sind.

3. Mehrwegeventil nach Anspruch 1, dadurch gekennzeichnet, daß die Membranen als Faltenbälge ausgebildet sind.

4. Mehrwegeventil nach einem der Ansprüche 1 bis 3, dadurch gekennzeichnet, daß die Membranen (4, 5) an zwei voneinander abgewandten Seiten des Ventilkörpers (1, 1', 12") angeordnet sind, und die Kopplungsvorrichtung als rechteckiger, die Ventilkammern (6, 7) umfassender Rahmen (8) ausgebildet ist, wobei die quer zur Bewegungsrichtung verlaufenden Teile (8a) des Rahmens jeweils mit den Außenseiten der Membranen (4, 5) verbunden sind und die in Bewegungsrichtung verlaufenden Teile (8b) des Rahmens durch seitliche Ausnehmungen oder Bohrungen (8d) des Ventilkörpers (1, 1', 12") geführt sind.

5. Mehrwegeventil nach einem der Ansprüche 1 bis 3, dadurch gekennzeichnet, daß die Membranen (4, 5) an der gleichen Seite des Ventilkörpers (21) angeordnet sind und die Kopplungsvorrichtung als zweiarmiger Hebel (28) ausgebildet ist, dessen Drehpunkt (28d) am Ventilkörper (21) gelagert ist und dessen Hebelarme (28a, 28b) jeweils mit den Außenseiten der Membranen (4, 5) verbunden sind und einer der Hebelarme (28a) mit der Betätigungsvorrichtung (13') verbunden ist.

6. Mehrwegeventil nach einem der Ansprüche 1 bis 3, dadurch gekennzeichnet, daß der Ventilkörper (31) U-förmig ausgebildet ist und die Membranen (4, 5) jeweils an der Innenseite der beiden Schenkel (31a, 31b) des Ventilkörpers (31) einander gegenüberliegend angeordnet sind, und die Kopplungsvorrichtung (38) zwischen den beiden Schenkeln (31a, 31b) des Ventilkörpers (31) angeordnet ist.

7. Mehrwegeventil nach Anspruch 6, dadurch gekennzeichnet, daß die Kopplungsvorrichtung als durchlaufende Verbindungsstange ausgebildet ist, an der die Betätigungsvorrichtung angreift.

8. Mehrwegeventil nach Anspruch 6, dadurch gekennzeichnet, daß die Betätigungsvorrichtung (10) zwischen den Schenkeln (31a, 31b) des Ventilkörpers (31) angeordnet ist und die Kopplungsvorrichtung (38) durch die Betätigungsvorrichtung (10) hindurchgeführt ist.

9. Mehrwegeventil nach Anspruch 8, dadurch gekennzeichnet, daß die Betätigungsvorrichtung eine Magnetspule (10) mit in Bewegungsrichtung der Membranen (4, 5) bewegbaren Anker (12") ist und der Anker (12") mit beiden Membranen (4, 5) verbunden ist.

10. Mehrwegeventil nach einem der Ansprüche 1 bis 3, dadurch gekennzeichnet, daß die Kopplungsvorrichtung ein hydraulisches System ist und jede der Membranen (4, 5) mit einem Hydraulikzylinder (44, 45) verbunden ist, wobei die beiden Hydraulikzylinder (44, 45) über eine Flüssigkeitsleitung (48, 49) miteinander verbunden sind und in die Flüssigkeitsleitung (48, 49) ein von außen betätigbarer, an seinen beiden Endflächen an die hydraulische Flüssigkeit angrenzender Steuerkolben (52) eingeschaltet ist.

11. Mehrwegeventil nach Anspruch 10, dadurch gekennzeichnet, daß der Steuerkolben (52) als Anker einer Magnetspule (10) ausgebildet ist.

12. Mehrwegeventil nach den Ansprüchen 10 oder 11, dadurch gekennzeichnet, daß die Hydraulikzylinder (44, 45) direkt am Ventilkörper (41) angeordnet sind und so ausgebildet sind, daß der Zylinder (44, 45) jeweils eine der Membranen (4, 5) an ihrer Außenseite umfaßt und der Zylinderkolben (46, 47) direkt an der Außenseite der Membran (4, 5) angeordnet ist.

13. Mehrwegeventil nach einem der Ansprüche 1 bis 4 oder 10 bis 12, dadurch gekennzeichnet, daß die beiden Ventilkammern über mehrere Verbindungskanäle miteinander verbunden sind, die um den Ventilsitz herum angeordnet sind.

14. Mehrwegeventil nach einem der Ansprüche 1 bis 12, dadurch gekennzeichnet, daß es als 3/2-Wegeventil ausgebildet ist, wobei in jeder der Ventilkammern (6, 7) ein mit einem Abflußkanal verbundener Ventilsitz (6a, 7a) angeordnet ist.

15. Mehrwegeventil nach einem der Ansprüche 1 bis 12, dadurch gekennzeichnet, daß es als 2/2-Wegeventil ausgebildet ist, wobei beide Membranen (4, 5) einen Ventilteller (4a, 5a) besitzen und in einer der Ventilkammern (6, 7) ein mit einem Abflußkanal verbundener Ventilsitz (7a', 6a'') angeordnet ist, während in der anderen Ventilkammer (6, 7) ein entsprechend dem Ventilsitz ausgebildeter geschlossener Aufsatz (6a', 7a'') angeordnet ist.

16. Mehrwegeventil nach Anspruch 15, dadurch gekennzeichnet, daß der Zuflußkanal (2', 2'') jeweils direkt mit der den geschlossenen Aufsatz (6a', 7a'') aufweisenden Ventilkammer verbunden ist.

17. Mehrwegeventil nach einem der Ansprüche 1 bis 16, dadurch gekennzeichnet, daß die Betätigungsvorrichtung eine Magnetspule (10) mit einem gegen Federkraft bewegbaren Anker (12, 12', 12'', 52) aufweist.

18. Mehrwegeventil nach einem der Ansprüche 1 bis 16, dadurch gekennzeichnet, daß die Betätigungsvorrichtung einen bewegbaren, zwei stabile Endlagen aufweisenden Dauermagneten enthält, sowie eine mit Schaltimpulsen beaufschlagbare Magnetspule zur Beförderung des Dauermagneten aus der einen in die andere Endlage.

19. Mehrwegeventil nach einem der Ansprüche 1 bis 18, dadurch gekennzeichnet, daß jeder Ventilteller (4a, 5a) an seiner dem Ventilsitz (6a, 7a) zugewandten Seite einen beim Schließen dieses Ventiltellers in den Ventilsitz (6a, 7a) eintauchenden Verschlußstopfen (4c, 5c) aufweist, dessen Durchmesser mindestens annähernd dem Innendurchmesser des Ventilsitzes (6a, 7a) entspricht, wobei die Länge dieser Verschlußstopfen und der Hub der Ventilteller (4a, 5a) so bemessen ist, daß in den beiden Endstellungen je ein Ventilsitz (6a, 7a) geöffnet ist, während in einer Mittelstellung beide Ventilsitze (6a, 7a) mindestens teilweise verschlossen sind.

**Claims**

1. A multiple-way valve, especially for use in dialyzers, comprising a valve body having inlet and outlet passages (2, 22, 32, 42 or 3a, 3b, 23a, 23b, 33a, 33b, 43a, 43b, respectively) provided therein, and having secured thereto two diaphragms (4, 5) of equal effective surface at the outer edges thereof in such a way that, between one surface of one of the diaphragms and one surface of the valve body, a valve chamber (6, 7) is formed, and that, on at least one of the diaphragms, a valve plate (4a, 5a) is arranged, said valve plate opposing a valve seat (6a, 7a) arranged in at least one of the valve chambers, through which valve seat said valve chamber is adapted to be connected with an outlet passage, whereby both diaphragms are connected with one another through a mechanic coupling device (8, 28, 38a, 48 to 52) which is rigid in its direction of movement, the connection being such that the diaphragms move in opposite directions relative to the valve chambers, and whereby an actuator (10) for displacing the diaphragms is provided, characterized in that the two valve chambers (6, 7) are connected with one another and with a common inlet passage (2, 22, 32, 42), that the diaphragms (4, 5) as well as the valve plate or plates (4a, 5a) are formed as closed components, that each of the valve plates (4a, 5a) is integral with the diaphragm associated therewith, and that the coupling device (8, 28, 38a, 48 to 52) is arranged externally of the valve chambers (6, 7) and of the inlet and outlet passages (2, 22, 32, 42 or 3a, 3b, 23a, 23b, 33a, 33b, 43a, 43b, respectively).

2. A multiple-way valve as claimed in claim 1, characterized in that the diaphragms are formed as roll-sliding diaphragms (4, 5).

3. A multiple-way valve as claimed in claim 1, characterized in that the diaphragms are formed as folded bellows.

4. A multiple-way valve as claimed in one of claims 1 to 3, characterized in that the diaphragms (4, 5) are arranged on two sides of the valve body (1, 1', 12'') facing away from one another, and that the coupling device is formed as a rectangular frame (8) enclosing the valve chambers (6, 7), whereby those components (8a) of the frame which extend transversely to the direction of movement, are each connected with the outer surfaces of the diaphragms (4, 5), and whereby those components (8b) of the frame which extend in the direction of movement, are passed through lateral recesses or bores (8d) of the valve body (1, 1', 12'').

5. A multiple-way valve as claimed in one of claims 1 to 3, characterized in that the diaphragms (4, 5) are arranged on the same side of the valve body (21), that the coupling device is formed as a double-armed lever (28) the fulcrum (28d) of which is positioned on the valve body (21), and the lever arms (28a, 28b) of which are each connected with the outer surfaces of the diaphragms (4, 5), and that one of the lever arms (28a) is connected with the actuator (13').

6. A multiple-way valve as claimed in one of claims 1 to 3, characterized in that the valve body (31) is U-shaped, that the diaphragms (4, 5) are each connected to the inner surfaces of the two legs (31a, 31b) of the valve body (31) in opposing relation to one another, and that the coupling device (38) is arranged between the two legs (31a, 31b) of the valve body (31).

7. A multiple-way valve as claimed in claim 6, characterized in that the coupling device is in the form of a continuous connecting bar which is acted upon by the actuator.

8. A multiple-way valve as claimed in claim 6, characterized in that the actuator (10) is arranged between the legs (31a, 31b) of the valve body (31), and that the coupling device (38) is passed through the actuator (10).

9. A multiple-way valve as claimed in claim 8, characterized in that the actuator includes a magnet coil (10) with an armature (12") which is displaceable in the direction of movement of the diaphragms (4, 5), and that the armature (12") is connected with both diaphragms (4, 5).

10. A multiple-way valve as claimed in one of claims 1 to 3, characterized in that the coupling device is a hydraulic system and each of the diaphragms (4, 5) is connected with a hydraulic cylinder (44, 45), whereby the two hydraulic cylinders (44, 45) are connected with one another through a fluid conduit (48, 49), and that a control piston (52) is inserted in the fluid conduit (48, 49) which control piston is adapted to be acted upon from the outside and is positioned, at its two end faces, adjacent to the hydraulic fluid.

11. A multiple-way valve as claimed in claim 10, characterized in that the control piston (52) is in the form of an armature of a magnet coil (10).

12. A multiple-way valve as claimed in claim 10 or 11, characterized in that the hydraulic cylinders (44, 45) are positioned directly adjacent to the valve body (41) and are formed in such a way that the cylinder (44, 45) encloses one each of the diaphragms (4, 5) on the outer surface thereof, and that the piston (46, 47) of the cylinder is arranged directly on the outer surface of the diaphragm (4, 5).

13. A multiple-way valve as claimed in claims 1 to 4 or 10 to 12, characterized in that both valve chambers are connected with one another through a plurality of connecting passages which are arranged around the valve seat.

14. A multiple-way valve as claimed in any of claims 1 to 12, characterized in that it is formed as a 3/2-way valve, whereby, in each of the valve chambers (6, 7), one valve seat (6a, 7a) connected with an outlet passage, is provided.

15. A multiple-way valve as claimed in any of claims 1 to 12, characterized in that it is formed as a 2/2-way valve, whereby both diaphragms (4, 5) comprise a valve plate (4a, 5a), and whereby a valve seat (7a, 6") connected with a outlet passage, is provided on one of the valve chambers (6, 7), whereas in the other valve chamber (6, 7), a closed cap (6a', 7a") corresponding, in its shape, to the valve seat, is provided.

16. A multiple-way valve as claimed in claim 15, characterized in that the inlet passage (2', 2") is directly connected with the valve chamber comprising the closed cap (6a', 7a).

17. A multiple-way valve as claimed in any of claims 1 to 16, characterized in that the actuator comprises a magnet coil (10) with an armature (12, 12', 12", 52) which is displaceable against the force of a spring.

18. A multi-way valve as claimed in any of claims 1 to 16, characterized in that the actuator comprises a movable permanent magnet having two stable end positions, and further comprises a magnet coil which is adapted to be acted upon by control pulses for shifting the permanent magnet from one end position into the other one.

19. A multiple-way valve as claimed in any of claims 1 to 18, characterized in that each valve plate (4a, 5a) comprises, on its side facing towards the valve seat (6a, 7a), a locking plug (4c, 5c) which, upon movement of this valve plate into the closed position, intrudes into the valve seat (6a, 7a), the diameter of said locking plug corresponding at least approximately to the inner diameter of said valve seat (6a, 7a), whereby the length of these locking plugs and the stroke of the valve plates (4a, 5a) are dimensioned so that, in both end positions, one each of the valve seats (6a, 7a) occupies its open position, whereas, in a medium position, both valve seats (6a, 7a) are at least partially closed.

**Revendications**

1. Vanne multi-voies en particulier destinée à l'utilisation sur des appareils à dialyse comportant un corps de vanne à travers lequel passent des canaux d'arrivée et de départ (2, 22, 32, 42 ou 3a, 3b, 23a, 23b, 33a, 33b, 43a, 43b) et sur lequel deux membranes (4, 5) à surface utile identique sont fixées par leurs bords de telle manière que respectivement entre une surface de l'une des membranes et une surface du corps de vanne se forme une chambre (6, 7) et que au moins sur l'une des membranes soit disposé un disque de vanne (4a, 5a) qui fait face à un siège de vanne (6a, 7a) disposé au moins dans une des chambres de vanne, siège au-dessus duquel cette chambre peut être reliée à un canal de sortie, les deux membranes étant reliées l'une à l'autre au moyen d'un dispositif d'accouplement (8, 28, 38a, 48, 52) mécanique, rigide dans le sens de déplacement des membranes, de telle manière que les membranes se déplacent en sens contraire par rapport aux chambres de vanne, un dispositif de commande (10) étant prévu pour le déplacement desdites membranes, caractérisée par le fait que les deux chambres de vanne (6, 7) sont reliées l'une à l'autre et à un canal d'arrivée commun (2, 22, 32, 42) et par le fait que aussi bien les membranes (4, 5) que les disques (4a, 5a) sont fermés et que chaque disque (4a, 5a) constitue une seule pièce avec la membrane qui lui correspond et que le dispositif d'accouplement (8, 28, 38a, 48 à 52) est disposé à l'extérieur des chambres (6, 7) et des canaux d'arrivée et de départ (2, 22, 32, 42 ou 3a, 3b, 23a, 23b, 33a, 33b, 43a, 43b).

2. Vanne multi-voies selon la revendication 1, caractérisée par le fait que les membranes sont réalisées sous la forme de membranes cylindriques (4, 5).

3. Vanne multi-voies selon la revendication 1, caractérisée par le fait que les membranes sont réalisées sous la forme de soufflets.

4. Vanne multi-voies selon l'une des revendications 1 à 3, caractérisée par le fait que les membranes (4, 5) sont disposées sur deux côtés écartées l'une de l'autre du corps de vanne (1, 1', 12") et que le dispositif d'accouplement est réalisé sous la forme d'un cadre (8) rectangulaire entourant les chambres (6, 7), les parties (8a) s'étendant transversalement par rapport au mouvement de déplacement du cadre étant respectivement reliées aux côtés externes des membranes (4, 5) et les parties (8b) du cadre s'étendant dans le sens du mouvement passant à travers des évidements latéraux ou des perçages (8d) pratiqués dans le corps de vanne (1, 1', 12").

5. Vanne multi-voies selon l'une des revendications 1 à 3, caractérisée par le fait que les membranes (4, 5) sont disposées sur le même côté du corps de vanne (21) et que le dispositif d'accouplement a la forme d'un levier à deux bras (28) dont le point de rotation (28d) s'appuie sur le corps de vanne (21) et dont les bras de levier (28a, 28b) sont reliés respectivement aux côtés extérieurs des membranes (4, 5) et que l'un des bras de levier (28a) est relié au dispositif de commande (13').

6. Vanne multi-voies selon l'une des revendications 1 à 3, caractérisée par le fait que le corps de vanne (31) est réalisé en forme de U et que les membranes (4, 5) sont disposées respectivement sur le côté interne des deux branches (31a, 31b) du corps de vanne (31) en se faisant face mutuellement, et que le dispositif d'accouplement (38) est disposé entre les deux branches (31a, 31b) du corps de vanne (31).

7. Vanne multi-voies selon la revendication 6, caractérisée par le fait que le dispositif d'accouplement est réalisé sous la forme d'une biellette passante sur laquelle s'articule le dispositif de commande.

8. Vanne multi-voies selon la revendication 6, caractérisée par le fait que le dispositif de commande (10) est disposé entre les branches (31a, 31b) du corps de vanne (31) et par le fait que le dispositif d'accouplement (38) passe à travers le dispositif de commande (10).

9. Vanne multi-voies selon la revendication 8, caractérisée par le fait que le dispositif de commande est une bobine magnétique (10) comportant un induit (12') mobile dans la direction du mouvement des membranes (4, 5) et par le fait que l'induit (12') est relié aux deux membranes (4, 5).

10. Vanne multi-voies selon l'une des revendications 1 à 3, caractérisée par le fait que le dispositif d'accouplement est un circuit hydraulique et que chacune des membranes (4, 5) est reliée à un cylindre hydraulique (44, 45), les deux cylindres hydrauliques (44, 45) étant reliés l'un à l'autre par une conduite de liquide (48, 49) et par le fait que dans cette conduite de liquide (48, 49) pénètre un piston de régulation (52) pouvant être commandé de l'extérieur dont les deux faces terminales sont limitrophes du liquide hydraulique.

11. Vanne multi-voies selon la revendication 10, caractérisée par le fait que le piston de régulation (52) est réalisé sous la forme d'un induit d'une bobine magnétique (10).

12. Vanne multi-voies selon les revendications 10 ou 11, caractérisée par le fait que les cylindres hydrauliques (44, 45) sont disposés directement sur le corps de vanne (41) et sont réalisés de telle manière que le cylindre (44, 45) entoure respectivement l'une des membranes (4, 5) sur son côté externe et que le piston du cylindre (46, 47) est disposé directement sur le côté externe de la membrane (4, 5).

13. Vanne multi-voies selon l'une des revendications 1 à 4, ou 10 à 12, caractérisée par le fait que les deux chambres de vanne sont reliées l'une à l'autre par plusieurs canaux de liaison qui sont disposés tout autour du siège de vanne.

14. Vanne multi-voies selon des revendications 1 à 12, caractérisée par le fait qu'elle est réalisée sous la forme d'une vanne à 3/2 voies, un siège de vanne (6a, 7a) relié à un canal de sortie étant disposé à l'intérieur de chacune des vannes.

15. Vanne multi-voies selon l'une des revendications 1 à 12, caractérisée par le fait qu'elle est réalisée sous la forme d'une vanne à 2/2 voies, deux membranes (4, 5) possédant un disque (4a, 5a) et un siège de vanne (7a', 6a") relié à un canal de sortie étant disposé à l'intérieur de l'une des chambres de vanne (6, 7), tandis que dans l'autre chambre de vanne (6, 7) est disposée une garniture fermée (6a', 7a') dont la forme correspond à celle du siège de vanne.

16. Vanne multi-voies selon la revendication 15, caractérisée par le fait que le canal d'arrivée (2', 2") est relié directement à la chambre respective présentant la garniture fermée (6a', 7a").

17. Vanne multi-voies selon l'une des revendications 1 à 16, caractérisée par le fait que le dispositif de commande présente une bobine magnétique (10) comportant un induit (12, 12', 12", 52) mobile dans le sens contraire à la force exercée par un ressort.

18. Vanne multi-voies selon l'une des revendications 1 à 16, caractérisée par le fait que le dispositif de commande contient un aimant permanent mobile présentant deux positions finales stables ainsi qu'une bobine magnétique pouvant être excitée par des impulsions de commande, en vue du transport de l'aimant permanent de l'une vers l'autre de ces deux positions finales.

19. Vanne multi-voies selon l'une des revendications 1 à 18, caractérisée par le fait que chaque disque (4a, 5a) présente sur son côté orienté vers le siège de vanne (6a, 7a) un bouchon d'obturation (4c, 5c) plongeant dans le siège (6a, 7a) lors de la fermeture de ce disque de vanne, bouchon dont le diamètre correspond au moins approximativement au diamètre interne du siège de vanne (6a, 7a), la longueur de ce bouchon et la course du disque (4a, 5a) étant dimensionnées de telle manière que dans les deux positions finales respectivement un siège (6a, 7a) est ouvert, tandis que dans une position centrale, les deux sièges (6a, 7a) sont au moins partiellement fermés.

FIG.1

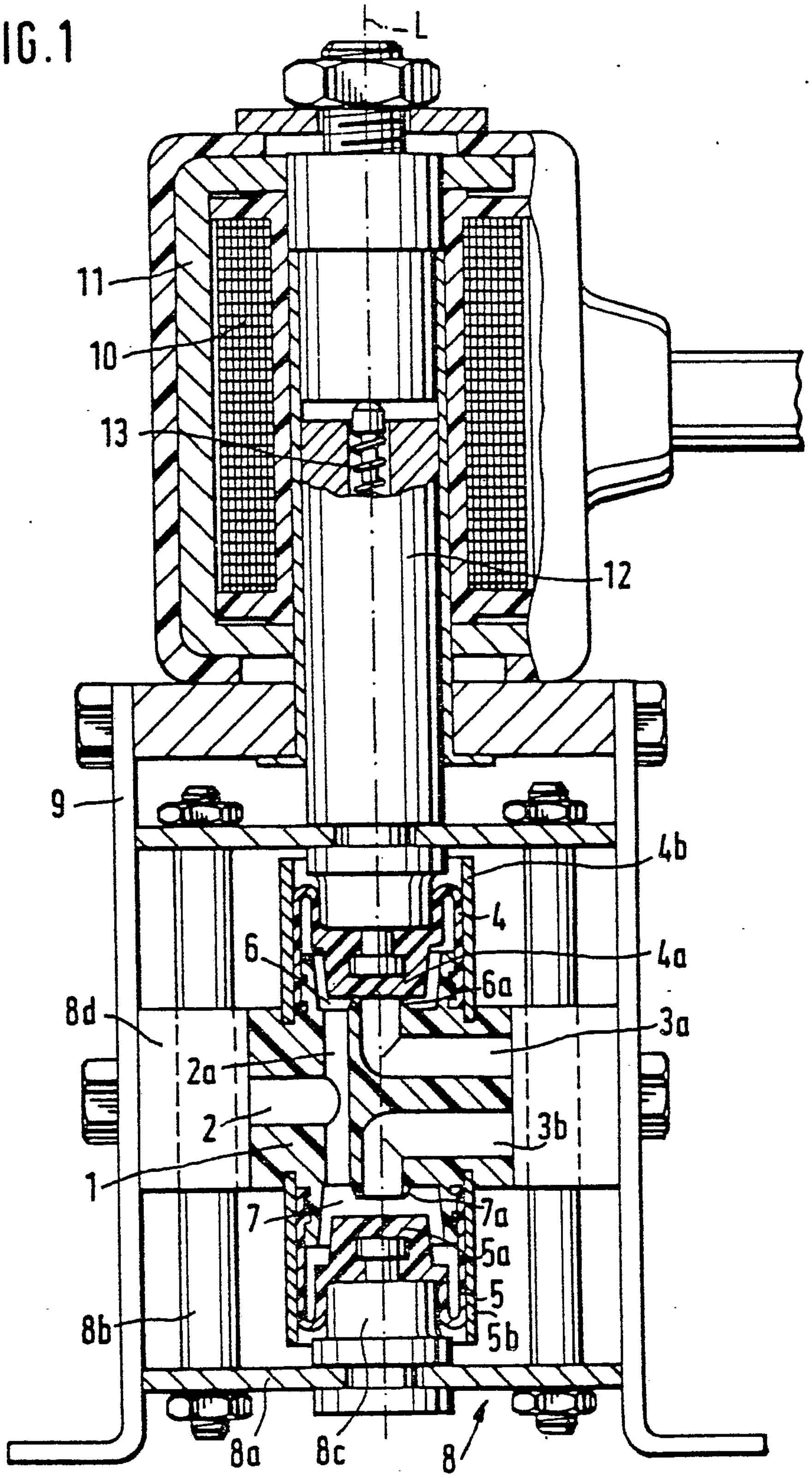

FIG. 2

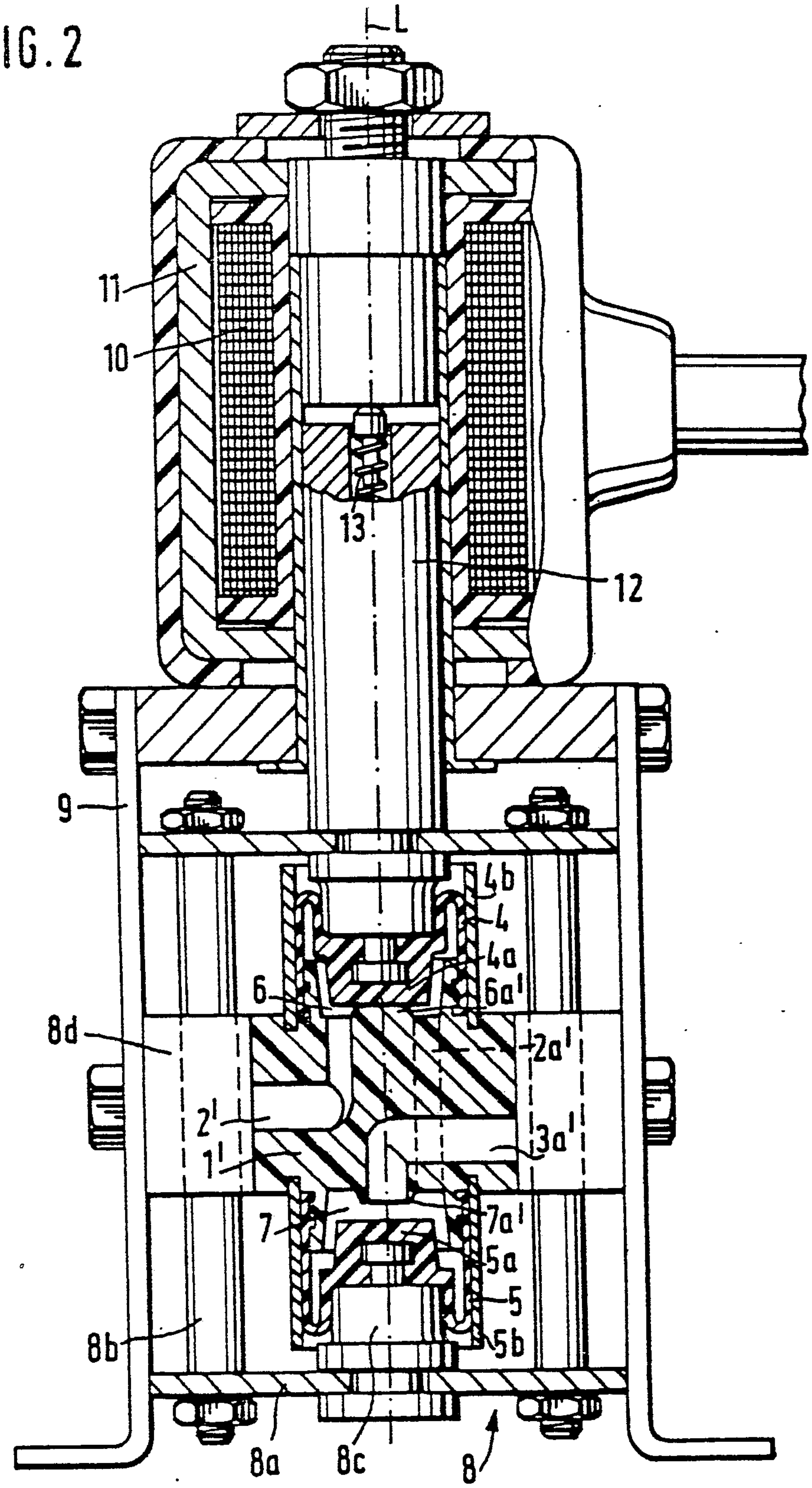

FIG. 3

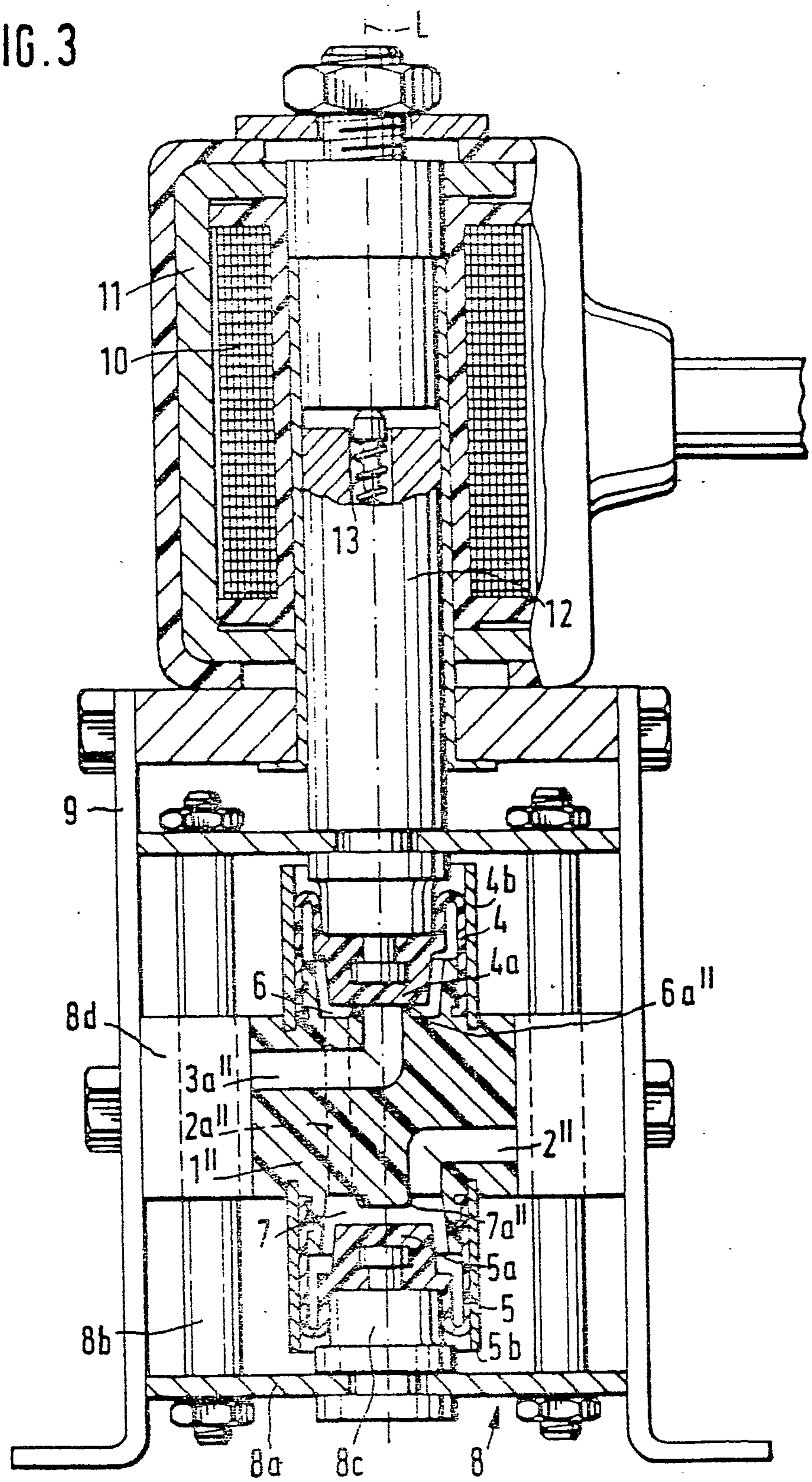

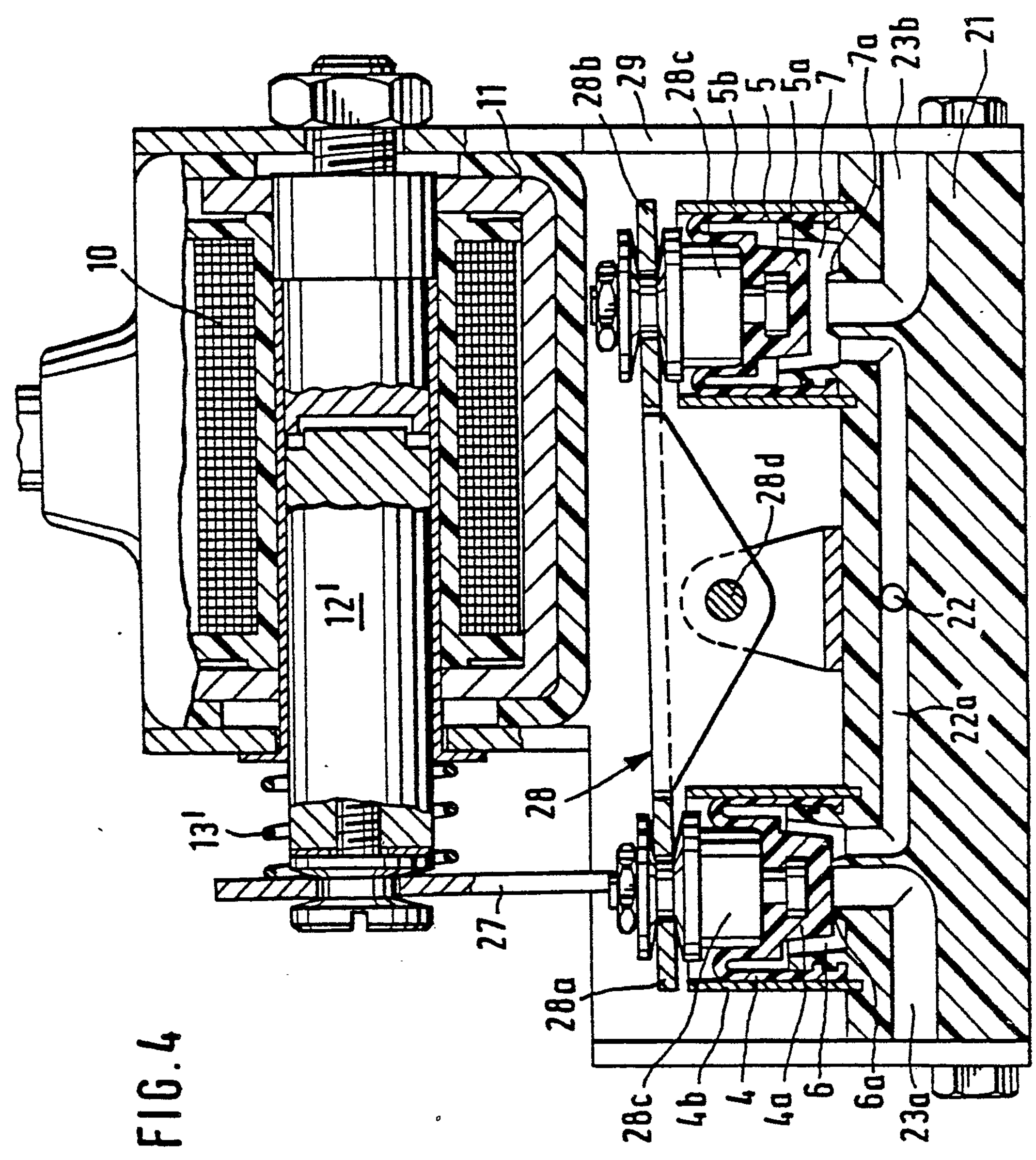
10
11
12'
13'
27
28
28a
28b
28c
28d
29
5b
5
5a
7
7a
23b
21
22
22a
4b
4
4a
6
6a
23a

FIG. 4

FIG.5

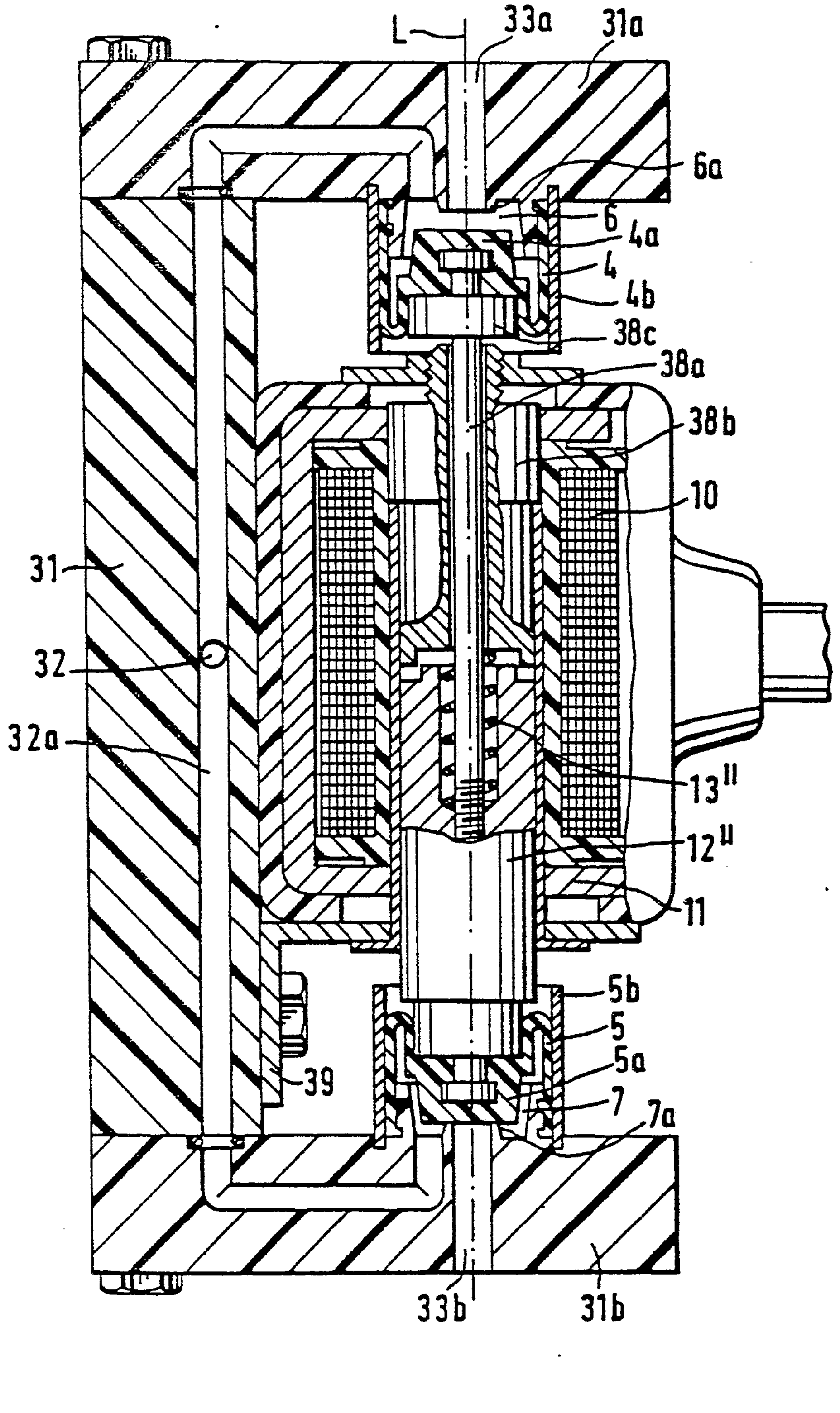
L
33a
31a
6a
6
4a
4
4b
38c
38a
38b
10
31
32
32a
13''
12''
11
5b
5
5a
39
7
7a
33b
31b

FIG.6

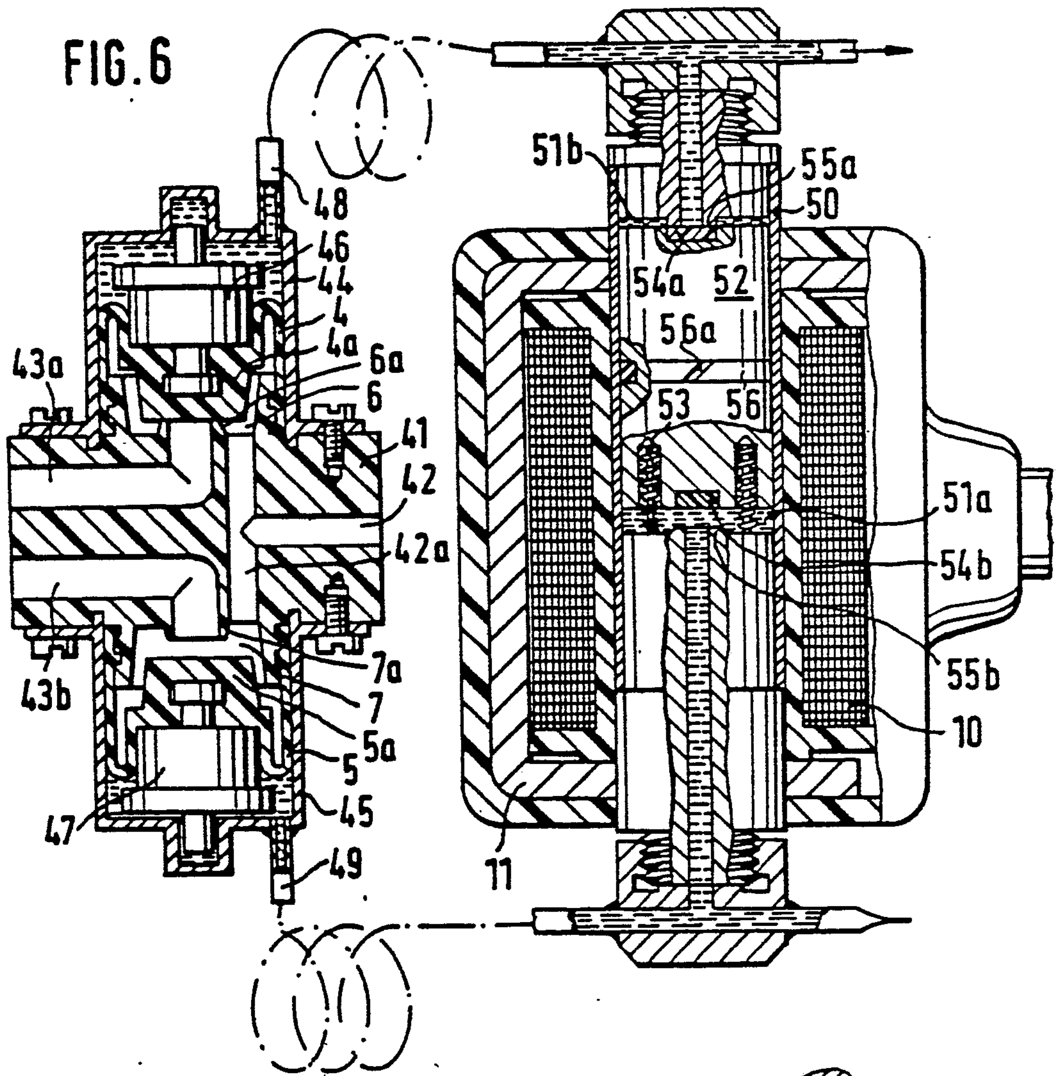
48
46
44
4
4a
6a
6
43a
41
42
42a
7a
7
43b
5a
5
45
47
49
51b
55a
50
54a
52
56a
53
56
51a
54b
55b
10
11

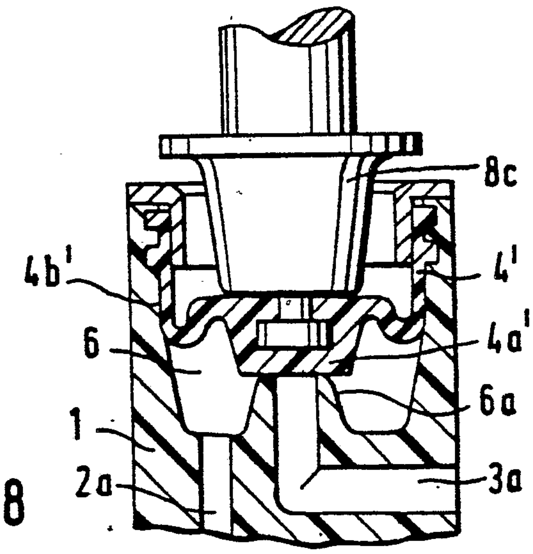
8c
4b'
4'
6
4a'
6a
1
2a
3a

FIG.8

FIG.7